# EUROPEAN PATENT SPECIFICATION

(11) **EP 4 257 095 B1**
(45) Date of publication and mention of the grant of the patent: **03.04.2024**
(21) Application number: 22166541.7
(22) Date of filing: 04.04.2022
(51) Int. Cl.: A61F 2/915

(54) **FLEXIBLE STENT AND METHOD FOR MANUFACTURING THE SAME**
FLEXIBLER STENT UND HERSTELLUNGSVERFAHREN DAFÜR
ENDOPROTHÈSE FLEXIBLE ET SON PROCÉDÉ DE FABRICATION

(43) Date of publication of application: 11.10.2023
(73) Proprietor: Sanita Biosciences BV, 3871 BL Hoevelaken (NL)
(72) Inventor: De Nooij, Gerrit, 3871BL Hoevelaken (NL); Fischer, Harald, 76356 Weingarten (DE); De Nooij, Jan Arend Thomas, 48940 Leioa (ES)
(74) Representative: Schornack, Oliver

(56) References cited:
- EP-A1- 0 895 759
- WO-A1-96/33671
- US-A1- 2005 187 610
- US-A1- 2007 067 011
- US-A1- 2014 364 935
- US-A1- 2019 021 809
- US-B2- 7 160 319

## Description

The present disclosure generally relates to the field of medical implants. More particularly, the present disclosure relates to a medical stent comprising at least two sections of cells, wherein each of the cells of one section is connected with one of the cells of the other section. The present disclosure further relates to a method for manufacturing such medical stent.

Medical stents are generally cylindrical devices which function to hold open and sometimes expand a segment of a blood vessel or other lumen such as a coronary artery. They are particularly suitable for use to support the lumen or hold back a dissected arterial lining which can occlude the fluid passageway therethrough.

A variety of devices are used as stents, such as a deformable structure in a variety of patterns that is expanded after being placed. They can be made as balloon expandable stents or as self-expandable stents. For instance, such stents may be helically wound coiled springs manufactured from an expandable metal and can be expanded by a balloon as exemplarily described in US 7,160,319 B2. Alternatively, such stents may be self-expanding stents inserted in a compressed state and shaped in a zigzag pattern, such as those described in US 2007/067011 A1 and US 2019/021809 A1.

For instance, US 2007/067011 A1 relates to a stent defined by a plurality of struts or other elements extending axially along and/or circumferentially around the stent in a zigzag or serpentine pattern. The zigzag pattern is defined by a plurality of axial elements that are substantially straight (extending substantially parallel to the longitudinal axis) connected alternately by curved elements extending about the circumference of the stent and is named a "band of cells". The length of the axial elements generally defines the axial length of the band of cells. A similar band of cells is connected to the (first) band of cells at adjacent peaks of the respective curved elements. According to one embodiment, variable mechanical properties along a length of the stent may be achieved by different sets of bands of cells. For instance, some of the bands of cells have zigzag patterns with a shorter period than others, so that they include more axial and curved elements providing greater luminal support and/or less flexibility. These bands of cells may be connected together using relatively short axial links, while other bands of cells are connected intermittently by longer diagonal links increasing flexibility.

US 2019/021809 A1 relates to a stent pattern including cylindrically aligned structural elements and linking structural elements. Depots for receiving a marker are located at a region of intersection of six structural elements. Alternatively, depots for markers are located at a region of intersection of four structural elements. The linking structural elements connect indent regions of a zigzag pattern of the cylindrically aligned structural elements in a longitudinal direction of the stent.

Furthermore, EP 0 895 759 A1 relates to a stent having a first portion (proximal segment in the implanted condition) and a second (generally distal) portion, wherein the first portion has a substantially more marked stiffness or resistance to radial compression stresses. The first portion can either have a denser mesh structure (the portion has a greater number of meshes per unit area), or by keeping the number of meshes per unit area in both portions identical, but providing wider wall portions or "braces" in the first portion. A transition between both portions may be achieved by means of a set of connecting bridges (links) extending generally axially relative to the stent.

One of the difficulties encountered width arterial stents involves maintaining the radial rigidity needed to hold open a body lumen while at the same time maintaining the flexibility of the stent to facilitate its delivery and accommodate the often tortuous path of the body lumen. Other problems encountered by using arterial stents involve maintaining stent flexibility and longitudinal stent compression. For example, these stents tend to experience high longitudinal stent compression when the stent is subject to an axial load, which can render the placement in the body lumen complicated.

It is thus an object of the present disclosure to provide a medical stent that facilitates placement of the stent in a body lumen and provides sufficient stability and to provide a method of manufacturing such stent.

This object is solved by the present invention as defined in the independent claims. Preferred embodiments are defined by the dependent claims.

According to a first aspect to better understand the present disclosure, a radially expandable stent comprises a first section arranged in a longitudinal direction of the stent, and a second section arranged in a longitudinal direction of the stent. The longitudinal direction of the stent is the axial direction, i.e., the direction along the tubular stent. The circumferential direction is along a cross-section of the stent, wherein the cross-section is made in a plane perpendicular to the longitudinal direction, such as along a circle or ellipse defined by the tubular stent. A radial direction means a direction perpendicular to the longitudinal axis of the stent and pointing to a surface of the stent. The radial direction is preferably arranged in the plane defined by the cross-section mentioned above.

The first section comprises a plurality of first cells arranged in a circumferential direction of the stent, wherein each of the plurality of first cells consists of a plurality of cell members, which together have a closed form. The plurality of first cells is connected at respective corners forming a peak of each first cell in the circumferential direction of the stent to form a ring of cells.

The first section or ring of first cells provides a first stent characteristic in a radial direction of the stent. For instance, such first stent characteristic can be a radial rigidity, radial expanding force, and/or compressibility in the radial direction (e.g., a ratio of a diameter or radius of the stent in a compressed form and an expanded form).

The second section comprises a plurality of second cells arranged in a circumferential direction of the stent, wherein each of the plurality of second cells consists of a plurality of cell members, which together have a closed form. The plurality of second cells is connected to one another in the circumferential direction of the stent to form a ring of cells. This second section provides a second stent characteristic in a radial direction of the stent. For instance, the second stent characteristic can be a radial rigidity, radial expanding force, and/or compressibility in the radial direction (e.g., a ratio of a diameter or radius of the stent in a compressed form and an expanded form). The second stent characteristic is different from the first stent characteristic, for example, a radial rigidity or radial expanding force may be lower for the second stent characteristic than for the first stent characteristic.

Furthermore, the stent comprises a plurality of first connectors, wherein each of the first cells is connected to at least one of the second cells by one of the first connectors. Thus, the ring of first cells and the ring of second cells is connected to one another in the longitudinal direction of the stent, wherein each cell of one ring is connected with at least one cell of the other ring by one first connector.

It is to be understood that the ring of first cells and the ring of second cells can be connected to one another by each cell of the ring of first cells being connected to one cell of the other ring by one first connector. Alternatively, one cell of the ring of first cells can be connected to two or more cells of the other ring by one first connector, such as a connector having more than two ends.

The plurality of first connectors provides a third stent characteristic in a longitudinal direction and/or a circumferential direction of the stent. For instance, the third stent characteristic can be a flexibility of the stent with respect to bending the longitudinal axis of the stent (e.g., allowing the stent to bend during delivery through a tortuous body lumen, such as a narrow and curved vessel). The third stent characteristic can additionally or alternatively be a compression strength in a longitudinal direction, e.g., a rigidity of the stent in the longitudinal direction. Also additionally or alternatively, the third stent characteristic can be a flexibility with respect to torsion of the stent, particularly torsion between the ring of first cells and the ring of second cells.

It is to be understood that the plurality of first connectors may also add to the first and/or second stent characteristic, since the first connectors also have an influence on the characteristics of the stent in the radial direction by combining both rings of cells.

The stent comprising at least two sections, each having a ring of connected cells, and connectors connecting the cells of both sections allow adaptation of the stent characteristics along the longitudinal direction of the stent. For instance, having different sections, the stent may be provided with different stent characteristics, such as the first to third stent characteristics, along the longitudinal direction of the stent. As an example only, a problematic area for an arterial stent is the flexibility in the stent's distal end. Many stents have uniform longitudinal flexibility along their length. However, a stent with a higher degree of flexibility in the distal end can better track through tortuous (and/or calcified) anatomy, and a stent with a lower degree of flexibility in the distal end can provide better rigidity at the respective end of the stent.

The stent further comprises a bar arranged at the respective corners forming the peak of each first cell in the circumferential direction of the stent to connect adjacent cells of the plurality of first cells to form the ring of cells. In other words, when viewed in the circumferential direction, adjacent cells are connected to one another with a bar.

In general, the disclosed stent allows optimal (longitudinal) flexibility for the intended medical indication as well as a sufficient radial force (radial rigidity). For instance, due to the optimal ratio of longitudinal flexibility to radial force, the disclosed stent can serve different medical indications to be treated with only one medical device, such as coil assisted stenting of an aneurysm, stenting of a vessel wall, e.g. to push a (rest) thrombus against the vessel wall, and stenting of vessel stenosis due to plaque and/or thrombus. The stent can be adapted to the particular medical indication/treatment by determining and/or setting the first to third characteristic of the stent in each of the sections and therebetween.

In a variant, the stent can further comprise a third section comprising a plurality of third cells connected to one another in the circumferential direction of the stent to form a ring of cells. Furthermore, the stent can comprise a plurality of second connectors, wherein each of the third cells is connected to at least one of the second cells by one of the second connectors. The plurality of second connectors provides a fourth stent characteristic in a longitudinal direction and/or a circumferential direction of the stent.

In another variant, the stent can further comprise a plurality of further sections each comprising a plurality of cells connected to one another in the circumferential direction of the stent to form a respective ring of cells, and a plurality of connectors. Each cell of a ring of cells can then be connected to at least one cell of a neighbouring ring of cells by one of the plurality of connectors. This allows a varying stent characteristic along the longitudinal direction. For instance, the stent can have a different radial rigidity at each end compared to a middle portion of the stent. Likewise, the stent can have a different longitudinally and/or circumferentially compressibility at one or both ends compared to a middle portion of the stent.

As an example only, the connectors arranged between rings of cells at an end of the stent can have a shorter length than connectors arranged between rings of cells at a middle part of the stent. These shorter connectors decrease flexibility in this section (portion) of the stent, while the longer connectors in the middle part of the stent increase flexibility. Particularly, the flexibility of the longitudinal axis of the stent is influenced by the length of the connectors. In other words, the stent becomes stiffer and less flexible at the respective ends of the stent, and becomes more flexible in the middle part. It is to be understood that the connectors of different lengths may also be provided at one or both ends of the stent, while the middle portion of the stent is provided with shorter connectors depending on the intended characteristics of the stent.

As another example, the connectors arranged between rings of cells can have varying thicknesses. For instance, if the stent requires a portion having a higher flexibility, thinner connectors can be provided between adjacent sections (i.e., adjacent rings of cells), while thicker connectors can be arranged between adjacent sections (rings of cells) where a higher rigidity of the stent is required.

Thus, the stent can have varying characteristics along the longitudinal direction and in the longitudinal direction and/or circumferential direction and/or radial direction. Such stent can be made in an optimal manner for the intended medical indication. This allows adaptation of the stent to the patient's anatomy and/or the intended medical treatment.

The stent comprises at least two sections, each comprising a plurality of cells connected to one another in the circumferential direction of the stent to form a respective ring of cells, wherein the cells of each ring are connected with the cells of a neighbouring ring of cells.

A cell is or consists of a plurality of cell members, which together have a closed form. Such closed form is best visible when viewing the stent from a side, for example, looking in a radial direction. As an example only, the first cell and/or the second cell may be a diamond cell or a form generally describing a rhomp and/or trapezoid or a combination of at least some these shapes. Of course, a cell can also have the shape of a circle, ellipse or polygon different from a rhomb or trapezoid or a combination of at least some of these shapes. For instance, a cell can generally have a hexagon form.

As an example only, a cell can have a stretched hexagon form, i.e. where two opposed cell members are longer than the remaining cell members. The longer cell members can, for example, be arranged substantially parallel to a longitudinal direction of the stent. Such elongated cell can be suitable for a vessel that has an opening, such as a secondary vessel or aneurysm. The stent can then be placed, so that the elongated cell is arranged at the opening. This allows access through the stent and to the opening. This is also particularly advantageous for example in case of a stent-assisted coiling of an aneurysm. Particularly, a coil can be guided in an easier manner through a larger cell, i.e., a large opening in the stent, than a smaller cell.

Furthermore, the cells of at least two of these sections may have the same form/shape. Also neighbouring rings of cells may have cells of the same form/shape.

Depending on the type of connector connecting such rings of cells, the stent characteristic can be the same along the longitudinal direction over one or more rings of cells or can vary, particularly if different types of connectors are used to connect the rings of cells. As an example only, at least three neighbouring rings of cells of the same form/shape can be connected with different types of connectors between a first and second ring and between the second and a third ring.

In another variant, the first connectors and/or the second connectors and/or the further connectors can be substantially straight, have an S-shape, have a Z-shape, have a U-shape, have a V-shape or be meandering or have a combination of at least some of these shapes, and/or can be arranged substantially parallel to the longitudinal direction of the stent, and/or can be arranged at an angle to the longitudinal direction of the stent, and/or can have at least a portion arranged substantially parallel to a circumferential direction of the stent, and/or can have at least one forking end forming at least a portion of a corner of the respective cell (to which it is connected). A connector having an S-shape, a Z-shape, a U-shape, a V-shape, or a meandering connector allow extension in the longitudinal direction of the stent, for example, during bending of the longitudinal axis of the stent. These connectors allow increasing the distance between the cells of adjacent rings of cells, particularly at the outer side of the stent when being bent. Thus, flexibility of the stent in the longitudinal direction is increased in this part/section of the stent.

In yet another variant, the plurality of first connectors and/or the plurality of second connectors and/or the plurality of further connectors may comprise at least two different types of connectors in the circumferential direction. Particularly, at least two different shapes of connectors may be used to connect two adjacent rings of cells. In other words, one portion of a ring of cells in the circumferential direction may be connected to the adjacent portion of the adjacent ring of cells with a first type/shape of connector, while the remaining portion of the ring of cells in the circumferential direction is connected to the corresponding portion of the adjacent ring of cells by a second type/shape of connector. This provides for a particular stent characteristic to be achieved only in the respective portion of the stent. For example, when cut along the longitudinal direction of the stent, each cut portion of the stent can have a respective characteristic. If, for instance, the stent is to be arranged with one side thereof next to an aneurysm (sack), this side of the stent or a portion of the stent on this side may have connectors that provide increased flexibility and/or the capability of the stent to elongate when bent, while the opposite side of the stent has a higher rigidity.

In another variant, the plurality of second cells can be connected at at least one respective corner of each second cell facing to an adjacent second cell in the circumferential direction of the stent to form the ring of cells. In other words, the plurality of second cells can be connected to one another in the circumferential direction at one corner, such as a peak of the respective second cell in the circumferential direction.

In another variant, each of the plurality of second cells can comprise a cell member arranged between a pair of corners, which are each connected to the adjacent second cell in the circumferential direction of the stent. In other words, the neighbouring second cells (i.e., neighbouring in the circumferential direction) share a common cell member.

It is to be understood that the plurality of second cells can be connected to one another in the circumferential direction at more than one corner, such as two or more corners or other locations of the respective second cell in the circumferential direction.

Furthermore, a strut or link of each second cell connecting two of the corners/peaks may be present, which runs substantially parallel to a strut or link of the neighbouring second cell (i.e., neighbouring in the circumferential direction). This provides a good resistance against kinking, particularly if the second cells have a larger extension in the longitudinal direction than other cells of the stent.

In a further variant, each of the first cells (of the first section) is connected to one of the plurality of second cells (of the second section) by a first connector between a corner forming a peak of the first cell in the longitudinal direction of the stent and a corner forming a peak of the second cell in the longitudinal direction of the stent.

In yet a further variant, as indicated above, at least one of the first cells (of the first section) is connected to more than one of the plurality of second cells (of the second section) by a split connector, such as a connector having one end to the side of the first section and having at least two ends to the side of the second section. It is to be understood that such split connector can also be employed in the other longitudinal direction, i.e. connecting one of the second cells (of the second section) with more than one of the first cells (of the first section).

In another variant, the stent can be a self-expendable stent. In other words, the rings of cells can be compressed in a radial direction, for example, to be placed on a catheter and/or in a tube. Once the stent is at the correct location in the patient's vessel or body, the stent is released from the catheter and/or tube and widens in the radial direction, i.e. self-expands. It is to be understood that the self-expendable stent can also be capable of being compressed in a longitudinal direction and self-expands in the longitudinal direction.

In yet another variant, the stent can comprise a bar arranged at the respective corners forming the peak of each second cell in the circumferential direction of the stent to connect adjacent cells of the plurality of second cells to form the ring of cells. In other words, when viewed in the circumferential direction, adjacent cells are connected to one another with a bar. Such bar may be of the same material as the cell and/or can be integrated into the cell(s).

In a further variant, the stent can comprise one of the plurality of first or second connectors arranged at the respective corners forming the peak of each second cell in the circumferential direction of the stent to connect adjacent cells of the plurality of second cells to form the ring of cells. Thus, when viewed in the circumferential direction, adjacent cells are connected to one another with a connector. This allows adaptation of the stent characteristic in the circumferential direction and/or a radial direction, such as provision of a certain flexibility and/or elongation of the ring of cells in the circumferential direction.

In another variant, the stent can further comprise at least one radiopaque marker. For example, the stent can comprise at least one tantalum marker. Such radiopaque marker provides excellent visibility when using x-ray or CT monitoring.

As an example only, the at least one radiopaque marker can be integrated into one of the first cells, one of the second cells or one of the connectors. Thus, the radiopaque marker can be provided anywhere in the stent, so that the entire stent or at least the most important section of the stent can be made visible.

In yet another variant, a plurality of radiopaque markers can be provided in the stent. For example, two radiopaque markers can be provided in the same section of the stent and opposite to one another in a radial direction. In other words, one radiopaque marker can be provided at a first location of the stent and a second radiopaque marker can be provided at a second location of the stent being at the same longitudinal position but in a radial direction on an opposite side of the stent.

This allows visibility of the stent at any time, by rotating the stent (e.g., while still being on the catheter) around the longitudinal axis of the stent.

In a further variant, the stent can further comprise at least one holding device configured to be coupled to a catheter. Such holding device can be provided at a longitudinal end of the stent. For instance, the holding device can be made from the same material that forms one of the cells. The holding device can be formed at a peak of one of such cells in the longitudinal direction. Moreover, the holding device can form a bulge or swelling in the circumferential direction and/or the radial direction.

In any case, the holding device can be configured to be coupled to a catheter, which can comprise a recess or the like having a negative form of the holding device. When the holding device is arranged in the negative form of the catheter, it can be covered by a tube or similar element of the catheter. Once the stent has reached the correct position in the patient's vessel or body lumen, the stent can be released from the catheter, while it self-expands. At the end having the holding device, the self-expendable stent can release itself from the negative form of the catheter by simply widening in the radial direction and thereby leaving the negative form of the catheter. This also allows pulling the stent back onto the catheter, i.e. under the tube, particularly if the stent is not fully released and the holding device is still coupled to the catheter. Thus, the stent can be repositioned in the vessel or body lumen, if the position appears not to be optimal.

In yet a further variant, a radiopaque marker can be provided in such holding device, for example, in the bulge or swelling formed by the holding device. Alternatively or additionally, a platinum or tantalum marker can be provided in such holding device.

In another variant, the stent can be made from Nitinol or stainless steel, such as CoCr or 316L. It is to be understood that the stent can be made from any material, which is suitable for a medical indication, such as being placed in a body vessel or body lumen and which provides the stent characteristics.

In yet another variant, the stent can have smoothed outer edges. Such smoothed outer edges avoid injury of the vessel wall and prevent traumas of the vessel or other body lumen. As an example only, the outer edges can be electro-polished.

According to a second aspect to better understand the present disclosure, a method for producing a stent comprises selecting a first type of cells, selecting a second type of cells, selecting a first type of connector, and manufacturing a stent according to the first aspect or one of its variants.

Specifically, the manufacturing of the stent includes arranging a plurality of first cells of the first type in a first section of the stent, arranging a plurality of second cells of the second type in a second section of the stent, connecting adjacent cells of the plurality of first cells with a bar at the respective corners forming the peak of each first cell in the circumferential direction of the stent, and connecting the first cells and the second cells with the first type of connector.

It is particularly advantageous if the first and second type of cells and the first type of connector are selected in view of the vessel or body lumen to be supported by the stent. Thus, an individual stent for each patient can be formed, that is particularly adapted to the circumstances of the patient's vessel, body lumen and/or anomaly. It is, of course, also possible to manufacture stents having specific stent characteristics that are often used for a plurality of patients and that suit a plurality of operations and treatments.

In a variant, the manufacturing comprises providing a tubular material for the stent, and cutting the tubular material according to the selected first and second type of cells and first type of connector. Thus, the sections and connectors of the stent can be made from one piece of material, i.e. can be manufactured in an integral manner. As an example only, laser cutting can be employed for cutting the tubular material.

In another variant, the method can further comprise providing at least one radiopaque marker, and integrating the at least one marker into one of the first cells, one of the second cells and/or one of the connectors. The integrating of the marker into the stent can take place, for example, by supplementing a radiopaque marker to the stent, once manufactured. For example, the marker can be adhered or welded to the stent, such as after cutting. As an alternative example, the marker is integrated into the tubular material before cutting the tubular material, while the cutting comprises cutting the tubular material around and at a (small) distance from the marker, so that the marker is surrounded by the material of the stent.

In yet another variant, the method can further comprise forming at least one holding device configured to be coupled to a catheter. Forming the holding device can, for example, include attaching the holding device to the stent, such as by adhering or welding. Alternatively, forming the holding device can be part of the manufacturing step, for example, the cutting step. In other words, the tubular material forming the stent is cut in such a manner that the holding device is formed.

In a further variant, the method can comprise selecting at least a third type of cells and/or at least one second type of connectors. The manufacturing can then comprise arranging a plurality of third cells of the third type in a third section of the stent, and connecting the second cells with the third cells with the second type of connector. Thus, a stent having varying stent characteristics along the longitudinal direction, i.e. in different sections, can be achieved.

Alternatively, the manufacturing can comprise arranging a plurality of third cells of the first or second type in a third section of the stent, and connecting the second cells with the third cells with the second type of connector. Thus, a stent can be achieved that has a longer portion (several sections) in the longitudinal direction having similar stent characteristics.

Further alternatively, the manufacturing can comprise arranging a plurality of third cells of the first, second or third type in a third section of the stent, and connecting the second cells with the third cells with the first type of connector. Likewise, this allows manufacturing a stent of similar stent characteristics in the longitudinal direction.

It is to be understood, that the method is not restricted to the above examples, but that an arbitrary number of sections with any combination of cell types, and connections between the sections with any connector type can be selected and a corresponding stent can be manufactured. Thus, the disclosed method allows the adaptation of the stent to any circumstances of the patients vessel, body lumen or anomaly.

The present disclosure is not restricted to the aspects and variants in the described form and order. Specifically, the description of aspects and variants is not to be understood as a specific limiting grouping of features. It is to be understood that the present disclosure also covers combinations of the aspects and variants not explicitly described. Thus, each variant or optional feature can be combined with any other aspect, variant, optional feature or even combinations thereof.

In the following, the present disclosure will further be described with reference to exemplary implementations illustrated in the figures, in which:
- Figure 1: illustrates an exemplary stent;
- Figure 2: illustrates an enlarged view of a first end and a second end of the stent of Figure 1;
- Figure 3: schematically illustrates a plurality of cells unreeled from a section of a stent;
- Figure 4: schematically illustrates a plurality of cells unreeled from another section of a stent;
- Figure 5: schematically illustrates a plurality of different connector types;
- Figure 6: schematically illustrates a plurality of cells unreeled from yet another section of a stent; and
- Figure 7: schematically illustrates a flow diagram of a method for producing a stent.

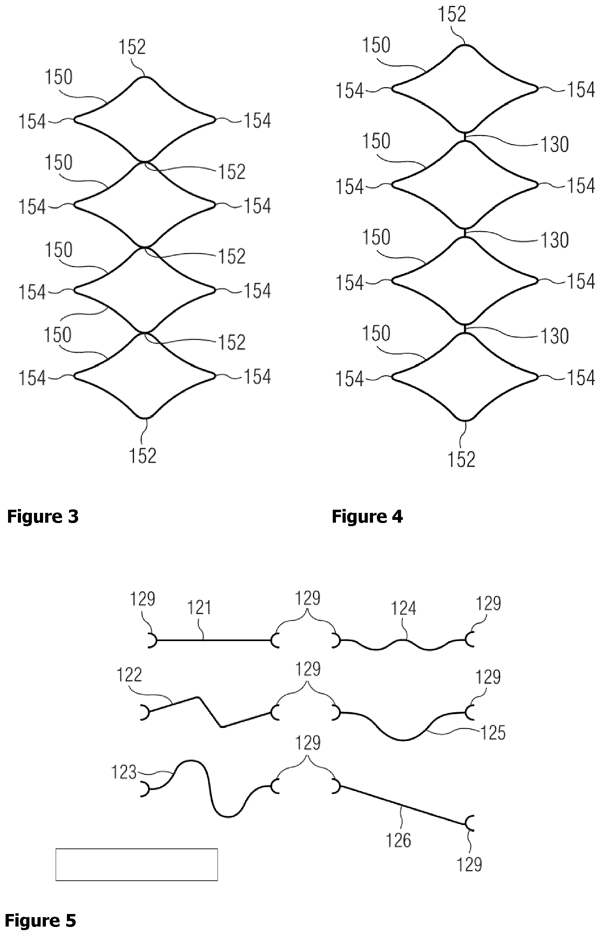

The configuration illustrated on fig. 3 is not covered by the present invention.

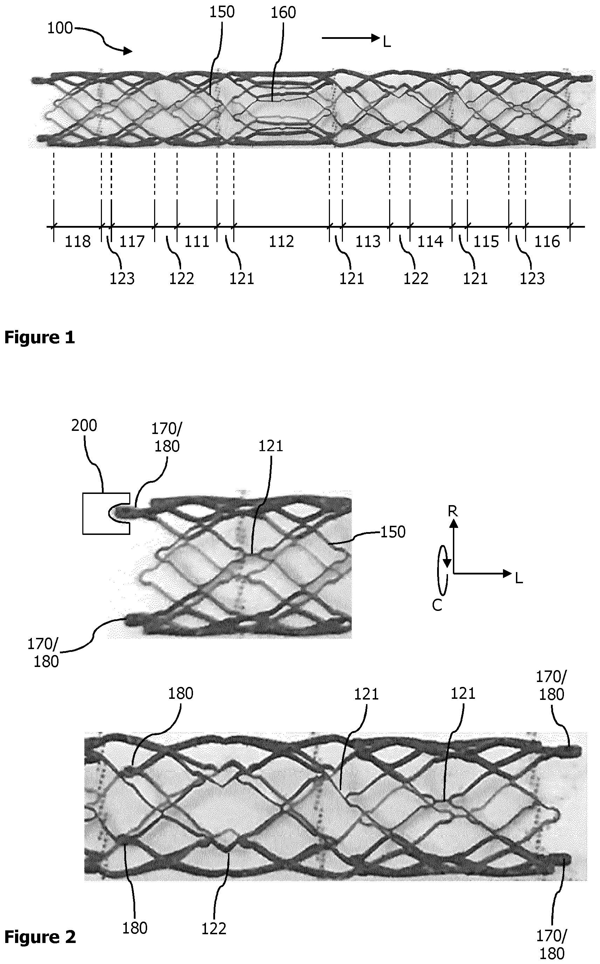

In the following description, for purposes of explanation and not limitation, specific details are set forth in order to provide a thorough understanding of the present disclosure. It will be apparent to one skilled in the art that the present disclosure may be practiced in other implementations that depart from these specific details. Figure 1 illustrates an exemplary stent 100, and Figure 2 illustrates enlarged views of the left end and right end of the stent 100. The stent 100 comprises at least a first section 111 and a second section 112 in a longitudinal direction L of the stent 100, i.e. the sections 111, 112 are arranged next to one another in the longitudinal direction of the stent 100. The first section 111 comprises a plurality of first cells 150 arranged in a circumferential direction C of the stent 100, and the second section 112 comprises a plurality of second cells 160 also arranged in the circumferential direction C of the stent 100. Each plurality of cells 150, 160 forms a ring of cells as will be explained in more detail with respect to Figures 3, 4 and 6.

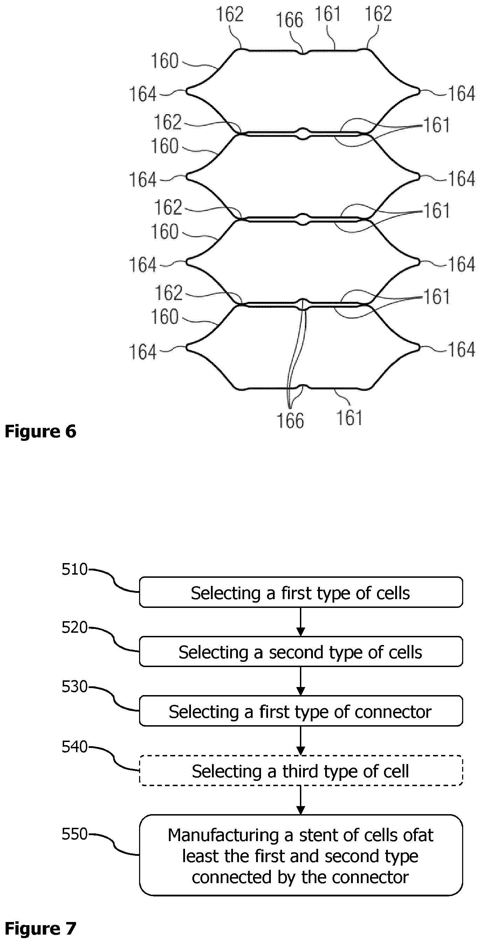

The stent comprises a plurality of sections, wherein eight sections 111 to 118 are illustrated in Figure 1. Each section 111 to 118 comprises a plurality of cells. Exemplary first cells 150 are illustrated in Figures 3 and 4 and exemplary second cells 160 are illustrated in Figure 6. All cells 150, 160 are depicted in an unreeled form in Figures 3, 4 and 6, while only four cells 150, 160 are shown. It is to be understood that the cells 150, 160 in Figures 3, 4 and 6 are arranged in the circumferential direction of the tubular stent 100, but have been unreeled from the stent 100. In other words, for the illustration of these drawings, the stent 100 is cut along the circumference C of the stent 100, in order to derive one section 111 to 118, and is cut in the longitudinal direction L. After unreeling the cut section, the row of cells 150, 160 of the respective drawing is achieved.

The plurality of first cells 150, as illustrated in Figure 3, is connected to one another at respective corners of each first cell 150. For example, the corners form a peak 152 of each first cell 150 in the circumferential direction C of the stent 100. The first cells 150 can, for example, be a diamond cell, i.e. have the shape of a trapezoid or rhomb. When viewing along the circumferential direction C, the diamond stands on-point, i.e. a respective peak 152 is pointing in each direction along the circumferential direction C. These peaks or corners 152 are the point of connection between two adjacent/neighbouring cells 150. Optionally, the corners 152 at the peaks in the circumferential direction C may not be sharp corners, but have a round or curved shape.

Each cell 150 further has a respective peak 154 in the longitudinal direction L of the stent 100. Optionally, the corners 154 at the peaks in the longitudinal direction L may not be sharp corners, but have a round or curved shape. For example, corner 154 may have a convex, concave and again convex course, where the concave portion forms the peak 154.

A curved corner 154 facilitates compressing of the stent 100 in the circumferential direction C and, hence, radial direction R, such as for placing a compressed stent 100 on a catheter 200 (partially shown in Figure 2). It is to be understood that a curved or round corner 152 at the circumferential outward peaks 152 also facilitate compressibility of the stent 100 in circumferential and radial direction C, R.

Each cell 150 can have a symmetric shape. For example, the cell 150 can be symmetric along a line parallel to the circumferential direction C, such as a line connecting the circumferentially outward peaks 152. In another example, cell 150 can be bisymmetric, for example along the circumferential direction C and the longitudinal direction L (i.e., can be symmetric with respect to the line connecting the circumferentially outward peaks 152 as well as with respect to a line connecting the longitudinally outward peaks 154).

Thus, each cell member of the cell 150, i.e., the cell member connecting a circumferentially outward peak 152 with a longitudinally outward peak 154, can be straight or curved. The cell members may be all of the same length, may be pairwise of same length (according to the symmetry of the cell 150), or may each be of a different length. In this disclosure a strut is interchangeable with cell member or link.

With respect to Figure 4, the plurality of first cells 150 can be the same as those illustrated in and described with respect to Figure 3. Thus, the description of the details of the cells 150 is omitted. The difference between the respective cells illustrated in Figures 3 and 4 is that two adjacent cells 150 in the circumferential direction C are connected to one another via a bar 130. The provision of such a bar 130, according to the present invention, provides a better radial rigidity of the ring of cells 150. On the other hand, if the cell size is selected smaller and the length of the bar 130 between two adjacent cells 150 is selected to be larger, the radial strength of the stent (the capability of the stent to provide a radial (outward force) is (slightly) decreased, but flexibility in the longitudinal direction of the stent is increased.

A different way of connecting cells 150 in the circumferential direction C can be achieved by arranging a connector 121 to 126 (Figure 5), instead of the bar 130. Thus, stent characteristics in the radial direction R, circumferential direction C as well as longitudinal direction L can be achieved by selecting a corresponding connector 121 to 126.

With references to Figure 6, a plurality of second cells 160 are illustrated. The second cells 160 are also unreeled as described above, while Figure 6 illustrates exemplarily four second cells 160.

The second cells 160 are each connected at at least one respective corner 162 facing an adjacent second cell 160 in the circumferential direction of the stent 100. In more detail, the second cells 160 have a generally hexagonal shape with two corners forming peaks 164 in a longitudinal direction of the stent 100 and four corners 162 at circumferentially outward edges 161 of the cell 160. For instance, a circumferentially outward cell member 161 may delimit the cell 160 in the circumferential direction C. At each end of this cell member 161, a corner 162 is formed, which also forms the connecting point to an adjacent cell 160 in the circumferential direction C to form the ring of cells. It is to be understood that the corners 162 may not necessarily be sharp corners, but can be round or curved.

In the illustrated example of the ring of cells 160, each cell 160 has two circumferentially outward edges/cell members 161, so that two cell members 161, one of each adjacent cell 160, are arranged substantially parallel to one another, while their respective ends, i.e., the respective corners 162, of both cells 161 are connected. This arrangement of two parallel cell members 161 allows bending the stent 100 along the longitudinal direction (i.e., the longitudinal axis of the stent 100 being bent) without kinking. For instance, a stent 100, such as the exemplary stent of Figure 1, can be bent by at least 90° without any kinking of the stent 100.

In the illustrated example, the second cells 160 are connected directly at their respective corners 162. It is to be understood that an optional arrangement may include at least one bar (not illustrated) connecting the corners 162 of the adjacent cells 160, such as bar 130 in Figure 4.

The generally hexagonal shape of the second cells 160 provide a larger opening in the stent 100 once placed in the patient's vessel or body lumen. This larger opening allows access to an opening in the vessel or body lumen, such as a secondary vessel or aneurysm, if the stent 100 is placed in the vessel or body lumen with the second cells 160, i.e., the second section 112 of the stent 100, at the opening in the vessel or body lumen.

In order to increase flexibility of the stent 100, the circumferentially outward cell members 161 may be provided with a dent 166 or short curved portion. In order to influence the stent characteristics in the longitudinal direction L, both cell members 161 may be provided with such dent 166, or only one of the two cell members 161 being arranged directly next to each other can be provided with such dent 166.

Turning back to Figures 1 and 2, the first to third stent characteristics may vary along the longitudinal direction L of the stent 100, i.e., vary from section to section, based on the connector 121 to 126 connecting the respective sections 111 to 118. For example, between the first section 111 and the second section 112 a first connector 121 is provided that is straight but arranged at an angle with respect to the longitudinal direction L. Between the first section 111 and the seventh section 117 there is arranged a V-shaped connector 122. Such V-shaped connector 122 is also provided between the third section 113 and the fourth action 114.

It is to be understood, that the connectors 121 to 126 between two adjacent sections 111 to 118 may be all the same. Alternatively, at least two different types of shapes of connectors 121 to 126 may be arranged between two adjacent sections 111 to 118. For example, with reference to Figure 3, one or more of the illustrated upper cells 150 may be connected to a neighbouring cell of the neighbouring section 111 to 118 with a first connector (a first type/shape of connector) 121 to 126, while the remaining (illustrated lower) cells are connected to the neighbouring cells of the neighbouring section 111 to 118 by a second connector (a second type/shape of connector) 121 to 126. Thus, the stent 100 is provided with different connectors 121 to 126 along the circumferential direction C, so that different stent characteristics can be achieved between two sections. For example, a flexibility of the stent 100 when its longitudinal axis L is bend can be influenced by different types of connectors 121 to 126 arranged in circumferential direction. With reference to Figure 1, the upper half of the illustrated stent 100 may have different stent characteristics than the lower half, if one half of the cells is connected with one type/shape of connector 121 to 126 and the other half of the cells is connected with another type/shape of connector 121 to 126.

Referring to Figure 5, a plurality of different types of connectors 121 to 126 are schematically illustrated. For instance, straight connectors 121, 126 may be provided, which can be arranged parallel to the longitudinal direction L (cf. connector 121) or at an angle to the longitudinal direction L (cf. connector 126). A further example is a bent connector 122, which consists mainly of straight segments with (sharp) corners therebetween. Further bent connectors, but rather curved connectors, can include an S-shaped connector 123, a waveform connector 124 and a rounded V-shaped connector 125.

Such bent or curved connectors 122 to 125 facilitate compressibility of the stent 100 in the longitudinal direction L as well as in the radial and circumferential direction R, C. The length of the connector influences the flexibility of the stent 100 of the longitudinal axis.

Likewise, the thickness of the respective connector 121 to 126 influences the flexibility of the stent 100 of the longitudinal axis. For instance, each of the connectors 121 to 126 may further be provided with different thicknesses, so that the stent characteristics can be optimized for the patient.

It is to be understood that this list of exemplary connectors 121 to 126 is not limiting and other forms and shapes of the connectors, other lengths further connectors 121 to 126 and/or varying thicknesses of the connectors 121 to 126 are possible.

As indicated above, each of these connectors 121 to 126 can likewise be employed to connect the cells 150, 160 in the circumferential direction C.

Each end of the connectors 121 to 126 is depicted with a forking end 129. Such forking end 129 provides a corner (or round corner) for the respective cell. In other words, at each side of the forking end 129 a cell member of the respective cell begins. It is to be understood that the forking end 129 may be considered only for illustrative purposes, since the entire stent 100 can be integrally formed, so that an end of the connector 121 to 126 and the beginning of the respective cell cannot be fixedly defined. The illustrated connectors 121 to 126, hence, do not limit the present disclosure to connectors 121 to 126 having a forking and 129. Rather, each of the connectors 121 to 126 can have a single pointed end.

Again with reference to Figure 2, the stent 100 can also comprises at least one radiopaque marker 180. Such marker 180 can be provided at a cell 150 or a connector 121 to 126, as is derivable from the lower portion of Figure 2. Such radiopaque marker 180 facilitate visibility of the stent when being placed, such as under x-ray and/or CT technology. For instance, the one or more markers 180 can be arranged at a second cell 160 and/or connector 121 to 126 close to the second cell 160, i.e., the second section 112. Thus, it is easily derivable for the operator where the second cell 160 is, for example, when it has to be placed over the opening of a secondary vessel or aneurysm.

In Figure 2 a plurality of radiopaque markers 180 is further illustrated as being provided in a holding device 170 arranged at each end of the stent 100. Such holding device 170 can be configured to be coupled to a catheter 200 (only partially illustrated), where the stent is releasably mounted to the catheter 200. The portion of the catheter 200 schematically illustrated in Figure 2 as a negative form corresponding to the holding device 170. For example, the negative form and catheter 200 may be arranged in such a manner that the holding device 170 is free to move in a radial direction R. This allows release of the stent 100 due to the self-expanding capabilities of the stent 100. In order to hold the compressed stent 100 in the catheter 200, a tube or ring (not illustrated) may cover the holding device 170 in a radial direction R during placement of the stent 100 and the tube or ring is removed, when the stent 100 is at the correct location. Once placed inside the body of the patient, the stent 100 can be released from the catheter 200. It is to be understood that each of the illustrated holding devices 170 may be arranged in a portion of the catheter 200, while Figure 2 only illustrates one of such portions for the sake of brevity. A radiopaque marker 180 provided in such holding device 170 facilitate detecting the beginning and end of the stent 100.

Optionally, the catheter 200, particularly, its portion holding the holding device 170 can be provided with a marker, such as marker 180. This facilitates introduction of the stent 100 into the patient's vessel and/or body lumen.

Figure 7 schematically illustrates a flow diagram of a method for producing a stent 100. The method includes a step 510 of selecting a first type of cells 150, and a step 520 of selecting a second type of cells 160. Furthermore, in step 530, a first type of connector 121 is selected.

In an optional step 540, a third type of cells can be selected. This optional step 540 may alternatively or additionally include selecting at least one second type of connector 122.

In step 550, the stent 100 is manufactured by arranging a plurality of first cells 150 of the first type in a first section 111, arranging a plurality of second cells 160 of the second type in a second section 112, and connecting the first cells 150 and the second cells 160 with a plurality of connectors 121 of the first type. It is to be understood that manufacturing the stent 100 can optionally include arranging a plurality of third cells in a third section 113 and connecting the third cells to the cells 150, 160 of the first or second section 111, 112.

The manufacturing 550 may be performed by laser cutting a tubular basic form, so that arranging cells and connecting the cells with connectors is actually performed during such laser cutting.

It is to be understood that the selecting steps 510 to 540 can be performed for an individual patient, i.e. after having knowledge of the required stent characteristics. The selecting steps 510 to 540 can even be performed shortly before the manufacturing step 550, such as a short time period before the laser cutting. A stent 100 can then be manufactured that is individually produced for a patient. Shortly before is not limited to a particular timespan, but can be a day before the operation (placement of the stent) or even a few minutes before the operation. Thus, the disclosed method allows manufacturing of a stent individualised for the patient as well as the location in the patient's vessel and/or body lumen.

It is believed that the advantages of the technique presented herein will be fully understood from the foregoing description, and it will be apparent that various changes may be made in the form, constructions and arrangement of the exemplary aspects thereof without departing from the scope of the disclosure or without sacrificing all of its advantageous effects. Because the technique presented herein can be varied in many ways, the invention is limited only by the scope of the claims that follow.

## Claims

1. A medical stent (100) comprising:
a first section (111) arranged in a longitudinal direction of the stent (100), the first section (111) comprises a plurality of first cells (150) arranged in a circumferential direction of the stent (100), wherein each of the plurality of first cells (150) consists of a plurality of cell members, which together have a closed form, wherein the plurality of first cells (150) is connected at respective corners forming a peak (152) of each first cell (150) in the circumferential direction of the stent (100) to form a ring of cells, and wherein the first section (111) provides a first stent characteristic in a radial direction of the stent (100);
a second section (112) arranged in the longitudinal direction of the stent (100), the second section (112) comprises a plurality of second cells (160) arranged in a circumferential direction of the stent (100), wherein each of the plurality of second cells (150) consists of a plurality of cell members, which together have a closed form, wherein the plurality of second cells (160) is connected to one another in the circumferential direction of the stent (100) to form a ring of cells, and wherein the second section (112) provides a second stent characteristic in the radial direction of the stent (100), wherein the first stent characteristic is different from the second stent characteristic; and
a plurality of first connectors (121), wherein each of the first cells (150) is connected to at least one of the second cells (160) by one of the first connectors (121), and wherein the plurality of first connectors (121) provides a third stent characteristic in a longitudinal direction and/or a circumferential direction of the stent (100),
**characterized in that** a bar (130) is arranged at the respective corners forming the peak (152) of each first cell (150) in the circumferential direction of the stent (100) to connect adjacent cells (150) of the plurality of first cells (150).

2. The stent (100) according to claim 1, further comprising:
a third section (113) comprising a plurality of third cells connected to one another in the circumferential direction of the stent (100) to form a ring of cells, wherein each of the plurality of third cells consists of a plurality of cell members, which together have a closed form; and
a plurality of second connectors (122), wherein each of the third cells is connected to at least one of the second cells (160) by one of the second connectors (122), wherein the plurality of second connectors (122) provides a fourth stent characteristic in a longitudinal direction and/or a circumferential direction of the stent (100).

3. The stent (100) according to claim 2, further comprising:
a plurality of further sections (111-118) each comprising a plurality of cells connected to one another in the circumferential direction of the stent (100) to form a respective ring of cells, wherein each of the plurality of cells consists of a plurality of cell members, which together have a closed form; and
a plurality of connectors (121-126), wherein each cell of a ring of cells is connected to at least one cell of a neighbouring ring of cells by one of the plurality of connectors (121-126),
wherein the connectors (121-126) arranged between rings of cells at an end of the stent (100) have a shorter length than connectors (121-126) arranged between rings of cells at a middle part of the stent (100).

4. The stent (100) according to one of claims 1 to 3, wherein the first connectors (121) and/or the second connectors (122):
are substantially straight, have an S-shape, have a Z-shape, have a U-shape, have a V-shape or are meandering, and/or
are arranged substantially parallel to the longitudinal direction of the stent (100), or are arranged at an angle to the longitudinal direction of the stent (100), and/or
have at least a portion arranged substantially parallel to a circumferential direction of the stent (100), and/or
have at least one forking end (129) forming at least a portion of a corner of the respective cell.

5. The stent (100) according to one of claims 1 to 4, wherein the plurality of second cells (160) is connected at at least one respective corner (162) of each second cell (160) facing to an adjacent second cell (160) in the circumferential direction of the stent (100) to form the ring of cells.

6. The stent (100) according to claim 5, wherein the plurality of cell members of each of the plurality of second cells (160) comprises a strut (161) arranged between a pair of corners, which are each connected to the adjacent second cell (160) in the circumferential direction of the stent (100).

7. The stent (100) according to claim 5 or 6, wherein a bar (130) or one of the plurality of first connectors (121) or one of the plurality of second connectors (122) is arranged at the respective corners of each second cell (160) in the circumferential direction of the stent (100) to connect adjacent cells (160) of the plurality of second cells (160).

8. The stent (100) according to one of claims 1 to 7, further comprising:
at least one radiopaque marker (180),
wherein, preferably, the at least one radiopaque marker (180) is integrated into one of the first cells (150), one of the second cells (160) or one of the connectors (121, 122).

9. The stent (100) according to one of claims 1 to 8, further comprising:
at least one holding device (170) configured to be coupled to a catheter (200).

10. The stent (100) according to one of claims 1 to 9, wherein the stent is made from Nitinol or stainless steel.

11. A method for producing a stent (100), the method comprising:
selecting (510) a first type of cells (150);
selecting (520) a second type of cells (160);
selecting (530) a first type of connector (121); and
manufacturing (550) a stent (100) according to one of claims 1 to 10 by arranging the plurality of first cells (150) of the first type in the first section (111) of the stent (100), and arranging the plurality of second cells (160) of the second type in the second section (112) of the stent (100), and connecting adjacent cells (150) of the plurality of first cells (150) with a bar (130) at the respective corners forming the peak (152) of each first cell (150) in the circumferential direction of the stent (100), and connecting the first cells (150) and the second cells (160) with the first type of connector (121).

12. The method of claim 11, wherein manufacturing (550) comprises:
providing a tubular material for the stent (100); and
cutting the tubular material according to the selected first and second type of cells (150, 160) and first type of connector (121).

13. The method of claim 11 or 12, further comprising:
providing at least one radiopaque marker (180); and
integrating the at least one marker (180) into one of the first cells (150), one of the second cells (160) and/or one of the connectors (121, 122).

14. The method of one of claims 11 to 13, further comprising:
forming at least one holding device (170) configured to be coupled to a catheter (200).

15. The method of one of claims 11 to 14, further comprising:
selecting (540) at least a third type of cells and/or at least one second type of connectors (122),
wherein manufacturing (550) comprises:
arranging a plurality of third cells of the third type in a third section (113) of the stent (100), and connecting the second cells (160) with the third cells with the second type of connector (122), or
arranging a plurality of third cells of the first or second type in a third section (113) of the stent (100), and connecting the second cells (160) with the third cells with the second type of connector (122), or
arranging a plurality of third cells of the first, second or third type in a third section (113) of the stent (100), and connecting the second cells (160) with the third cells with the first type of connector (122).

## Patentansprüche

1. Medizinischer Stent (100), umfassend:
einen ersten Abschnitt (111), der in einer Längsrichtung des Stents (100) angeordnet ist, wobei der erste Abschnitt (111) eine Mehrzahl von ersten Zellen (150) umfasst, die in einer Umfangsrichtung des Stents (100) angeordnet sind, wobei jede der Mehrzahl von ersten Zellen (150) aus einer Mehrzahl von Zellelementen besteht, die zusammen eine geschlossene Form aufweisen, wobei die Mehrzahl von ersten Zellen (150) an jeweiligen Ecken, die eine Spitze (152) jeder ersten Zelle (150) in der Umfangsrichtung des Stents (100) bilden, verbunden ist, um einen Ring von Zellen zu bilden, und wobei der erste Abschnitt (111) eine erste Stentcharakteristik in einer radialen Richtung des Stents (100) bereitstellt;
einen zweiten Abschnitt (112), der in der Längsrichtung des Stents (100) angeordnet ist, wobei der zweite Abschnitt (112) eine Mehrzahl von zweiten Zellen (160) umfasst, die in einer Umfangsrichtung des Stents (100) angeordnet sind, wobei jede der Mehrzahl von zweiten Zellen (150) aus einer Mehrzahl von Zellelementen besteht, die zusammen eine geschlossene Form aufweisen, wobei die Mehrzahl von zweiten Zellen (160) in der Umfangsrichtung des Stents (100) miteinander verbunden ist, um einen Ring von Zellen zu bilden, und wobei der zweite Abschnitt (112) eine zweite Stentcharakteristik in der radialen Richtung des Stents (100) bereitstellt, wobei die erste Stentcharakteristik von der zweiten Stentcharakteristik verschieden ist; und
eine Mehrzahl von ersten Verbindern (121), wobei jede der ersten Zellen (150) mit mindestens einer der zweiten Zellen (160) durch einen der ersten Verbinder (121) verbunden ist, und wobei die Mehrzahl der ersten Verbinder (121) eine dritte Stentcharakteristik in einer Längsrichtung und/oder einer Umfangsrichtung des Stents (100) bereitstellt,
**dadurch gekennzeichnet, dass** ein Steg (130) an den jeweiligen Ecken, die die Spitze (152) jeder ersten Zelle (150) in der Umfangsrichtung des Stents (100) bilden, angeordnet ist, um benachbarte Zellen (150) der Mehrzahl von ersten Zellen (150) zu verbinden.

2. Stent (100) nach Anspruch 1, ferner umfassend:
einen dritten Abschnitt (113), der eine Mehrzahl von dritten Zellen umfasst, die miteinander in der Umfangsrichtung des Stents (100) verbunden sind, um einen Ring von Zellen zu bilden, wobei jede der Mehrzahl von dritten Zellen aus einer Mehrzahl von Zellelementen besteht, die zusammen eine geschlossene Form aufweisen; und
eine Mehrzahl von zweiten Verbindern (122), wobei jede der dritten Zellen mit mindestens einer der zweiten Zellen (160) durch einen der zweiten Verbinder (122) verbunden ist, wobei die Mehrzahl der zweiten Verbinder (122) eine vierte Stentcharakteristik in einer Längsrichtung und/oder einer Umfangsrichtung des Stents (100) bereitstellt.

3. Stent (100) nach Anspruch 2, ferner umfassend:
eine Mehrzahl von weiteren Abschnitten (111-118), die jeweils eine Mehrzahl von Zellen umfassen, die miteinander in der Umfangsrichtung des Stents (100) verbunden sind, um einen jeweiligen Ring von Zellen zu bilden, wobei jede der Mehrzahl von Zellen aus einer Mehrzahl von Zellelementen besteht, die zusammen eine geschlossene Form aufweisen; und
eine Mehrzahl von Verbindern (121-126), wobei jede Zelle eines Rings von Zellen mit mindestens einer Zelle eines benachbarten Rings von Zellen durch einen der Mehrzahl von Verbindern (121-126) verbunden ist,
wobei die Verbinder (121-126), die zwischen den Zellringen an einem Ende des Stents (100) angeordnet sind, eine kürzere Länge haben als die Verbinder (121-126), die zwischen den Zellringen in einem mittleren Teil des Stents (100) angeordnet sind.

4. Stent (100) nach einem der Ansprüche 1 bis 3, wobei die ersten Verbinder (121) und/oder die zweiten Verbinder (122):
im Wesentlichen gerade, S-förmig, Z-förmig, U-förmig, V-förmig oder mäanderförmig sind, und/oder
im Wesentlichen parallel zur Längsrichtung des Stents (100) angeordnet sind oder in einem Winkel zur Längsrichtung des Stents (100) angeordnet sind, und/oder
zumindest einen Abschnitt aufweisen, der im Wesentlichen parallel zu einer Umfangsrichtung des Stents (100) angeordnet ist, und/oder
mindestens ein gabelförmiges Ende (129) aufweisen, das mindestens einen Teil einer Ecke der jeweiligen Zelle bildet.

5. Stent (100) nach einem der Ansprüche 1 bis 4, wobei die Mehrzahl von zweiten Zellen (160) an mindestens einer jeweiligen Ecke (162) jeder zweiten Zelle (160), die einer benachbarten zweiten Zelle (160) in der Umfangsrichtung des Stents (100) zugewandt ist, verbunden ist, um den Ring von Zellen zu bilden.

6. Stent (100) nach Anspruch 5, wobei die Mehrzahl von Zellelementen jeder der Mehrzahl von zweiten Zellen (160) eine Strebe (161) umfasst, die zwischen einem Paar von Ecken, die jeweils mit der benachbarten zweiten Zelle (160) in der Umfangsrichtung des Stents (100) verbunden sind, angeordnet ist.

7. Stent (100) nach Anspruch 5 oder 6, wobei ein Steg (130) oder einer der Mehrzahl von ersten Verbindern (121) oder einer der Mehrzahl von zweiten Verbindern (122) an den jeweiligen Ecken jeder zweiten Zelle (160) in der Umfangsrichtung des Stents (100) angeordnet ist, um benachbarte Zellen (160) der Mehrzahl von zweiten Zellen (160) zu verbinden.

8. Stent (100) nach einem der Ansprüche 1 bis 7, ferner umfassend:
mindestens einen röntgendichten Marker (180),
wobei vorzugsweise der mindestens eine röntgendichte Marker (180) in eine der ersten Zellen (150), eine der zweiten Zellen (160) oder einen der Verbinder (121, 122) integriert ist.

9. Stent (100) nach einem der Ansprüche 1 bis 8, ferner umfassend:
mindestens eine Haltevorrichtung (170), die dazu eingerichtet ist, mit einem Katheter (200) verbunden zu werden.

10. Stent (100) nach einem der Ansprüche 1 bis 9, wobei der Stent aus Nitinol oder rostfreiem Stahl hergestellt ist.

11. Verfahren zur Herstellung eines Stents (100), wobei das Verfahren umfasst:
Auswählen (510) einer ersten Art von Zellen (150);
Auswählen (520) einer zweiten Art von Zellen (160);
Auswählen (530) einer ersten Art eines Verbinders (121); und
Herstellen (550) eines Stents (100) nach einem der Ansprüche 1 bis 10 durch Anordnen der Mehrzahl von ersten Zellen (150) des ersten Typs im ersten Abschnitt (111) des Stents (100) und Anordnen der Mehrzahl von zweiten Zellen (160) des zweiten Typs im zweiten Abschnitt (112) des Stents (100), und Verbinden benachbarter Zellen (150) der Mehrzahl von ersten Zellen (150) mit einem Steg (130) an den jeweiligen Ecken, die die Spitze (152) jeder ersten Zelle (150) in der Umfangsrichtung des Stents (100) bilden, und Verbinden der ersten Zellen (150) und der zweiten Zellen (160) mit der ersten Art von Verbinder (121).

12. Verfahren nach Anspruch 11, wobei das Herstellen (550) umfasst:
Bereitstellen eines röhrenförmigen Materials für den Stent (100); und
Schneiden des röhrenförmigen Materials entsprechend der ausgewählten ersten und zweiten Art von Zellen (150, 160) und der ersten Art von Verbinder (121).

13. Verfahren nach Anspruch 11 oder 12, ferner umfassend:
Bereitstellen mindestens eines röntgendichten Markers (180); und
Integrieren des mindestens einen Markers (180) in eine der ersten Zellen (150), eine der zweiten Zellen (160) und/oder einen der Verbinder (121, 122).

14. Verfahren nach einem der Ansprüche 11 bis 13, ferner umfassend:
Ausbilden mindestens einer Haltevorrichtung (170), die dazu eingerichtet ist, mit einem Katheter (200) verbunden zu werden.

15. Verfahren nach einem der Ansprüche 11 bis 14, ferner umfassend:
Auswählen (540) mindestens einer dritten Art von Zellen und/oder mindestens einer zweiten Art von Verbindern (122),
wobei das Herstellen (550) umfasst:
Anordnen einer Mehrzahl von dritten Zellen der dritten Art in einem dritten Abschnitt (113) des Stents (100), und Verbinden der zweiten Zellen (160) mit den dritten Zellen mit der zweiten Art von Verbindern (122), oder
Anordnen einer Mehrzahl von dritten Zellen der ersten oder zweiten Art in einem dritten Abschnitt (113) des Stents (100) und Verbinden der zweiten Zellen (160) mit den dritten Zellen mit der zweiten Art von Verbinder (122), oder
Anordnen einer Mehrzahl von dritten Zellen der ersten, zweiten oder dritten Art in einem dritten Abschnitt (113) des Stents (100) und Verbinden der zweiten Zellen (160) mit den dritten Zellen mit der ersten Art von Verbinder (122).

## Revendications

1. Endoprothèse médicale (100) comprenant:
une première section (111) disposée dans une direction longitudinale de l'endoprothèse (100), la première section (111) comprenant une pluralité de premières cellules (150) disposées dans une direction circonférentielle de l'endoprothèse (100), chacune de la pluralité des premières cellules (150) étant constituée d'une pluralité d'éléments de cellule, qui, ensemble, ont une forme fermée, la pluralité de premières cellules (150) étant reliées au niveau des coins respectifs formant un sommet (152) de chacune première cellule (150) dans la direction circonférentielle de l'endoprothèse (100) pour former un anneau de cellules, et la première section (111) fournissant une première caractéristique d'endoprothèse dans une direction radiale de l'endoprothèse (100);
une seconde section (112) disposée dans une direction longitudinale de l'endoprothèse (100), la seconde section (112) comprenant une pluralité de secondes cellules (160) disposées dans une direction circonférentielle de l'endoprothèse (100), chacune de la pluralité des secondes cellules (150) étant constituée d'une pluralité d'éléments de cellule, qui, ensemble, ont une forme fermée, la pluralité de secondes cellules (160) étant reliées l'une à l'autre dans la direction circonférentielle de l'endoprothèse (100) pour former un anneau de cellules, et la seconde section (112) fournissant une seconde caractéristique d'endoprothèse dans une direction radiale de l'endoprothèse (100), la première caractéristique d'endoprothèse étant différente de la seconde caractéristique d'endoprothèse; et
une pluralité de premiers connecteurs (121), dans laquelle chacune des premières cellules (150) est reliée à au moins l'une des secondes cellules (160) par l'un des premiers connecteurs (121), et la pluralité des premiers connecteurs (121) fournissant une troisième caractéristique d'endoprothèse dans une direction longitudinale et/ou dans une direction circonférentielle de l'endoprothèse (100),
**caractérisé en ce qu'**une barre (130) est disposée aux coins respectifs formant le sommet (152) de chaque première cellule (150) dans la direction circonférentielle de l'endoprothèse (100) pour relier des cellules adjacentes (150) de la pluralité de premières cellules (150).

2. Endoprothèse (100) selon la revendication 1, comprenant en outre:
une troisième section (113) comprenant une pluralité de troisièmes cellules reliées l'une à l'autre dans la direction circonférentielle de l'endoprothèse (100) pour former un anneau de cellules, chacune de la pluralité des troisièmes cellules étant constituée d'une pluralité d'éléments de cellule qui ensemble forment une forme fermée; et
une pluralité de seconds connecteurs (122), dans laquelle chacune des troisièmes cellules est reliée à au moins l'une des secondes cellules (160) par l'un des seconds connecteurs (122), et la pluralité des seconds connecteurs (122) fournissant une quatrième caractéristique d'endoprothèse dans une direction longitudinale et/ou dans une direction circonférentielle de l'endoprothèse (100).

3. Endoprothèse (100) selon la revendication 2, comprenant en outre:
une pluralité d'autres sections (111- 118) chacune comprenant une pluralité de cellules reliées l'une à l'autre dans la direction circonférentielle de l'endoprothèse (100) pour former un anneau respectif de cellules, dans laquelle chacun de la pluralité des éléments de cellules qui ensemble forment une forme fermée; et
une pluralité de connecteurs (121-126), dans lequel chaque cellule d'un anneau de cellules est reliée à au moins une cellule d'un anneau voisin de cellules par l'un parmi la pluralité de connecteurs (121-126),
dans lequel les connecteurs (121-126) disposés entre des anneaux de cellules à une extrémité de l'endoprothèse (100) ont une longueur inférieure aux connecteurs (121-126) disposés entre les anneaux de cellules dans une partie centrale de l'endoprothèse (100).

4. Endoprothèse (100) selon l'une quelconque des revendications 1 à 3, dans laquelle les premiers connecteurs (121) et/ou les seconds connecteurs (122):
sont sensiblement droits, sont en forme de S, sont en forme de Z, sont en forme de U, sont en forme de V ou sont des méandres, et/ou
sont sensiblement disposés parallèlement à la direction longitudinale de l'endoprothèse (100), ou sont disposés dans un angle par rapport à la direction longitudinale de l'endoprothèse (100), et/ou
ont au moins une partie disposée sensiblement parallèle à une direction circonférentielle de l'endoprothèse (100), et/ou
ont au moins une extrémité en forme de fourche (129) formant au moins une partie d'un coin de la cellule respective.

5. Endoprothèse (100) selon l'une quelconque des revendications 1 à 4, dans laquelle la pluralité de secondes cellules (160) est reliée à au moins un coin respectif (162) de chaque seconde cellule (160) faisant face à une seconde cellule adjacente (160) dans la direction circonférentielle de l'endoprothèse (100) pour former l'anneau de cellules.

6. Endoprothèse (100) selon la revendication 5, dans laquelle la pluralité d'éléments de cellule de chacune de la pluralité de secondes cellules (160) comprend une entretoise (161) disposée entre une paire de coins, qui sont chacun reliés à la seconde cellule adjacente (160) dans la direction circonférentielle de l'endoprothèse (100).

7. Endoprothèse (100) selon la revendication 5 ou 6, dans laquelle une barre (130) ou l'un parmi la pluralité de premiers connecteurs (121) ou l'un parmi la pluralité de seconds connecteurs (122) est disposé(e) aux coins respectifs de chaque seconde cellule (160) dans la direction circonférentielle de l'endoprothèse (100) pour relier des cellules adjacentes (160) de la pluralité de secondes cellules (160).

8. Endoprothèse (100) selon l'une quelconque des revendications 1 à 7, comprenant en outre:
au moins un marqueur radioopaque (180),
dans lequel, de préférence, l'au moins un marqueur radioopaque (180) est intégré dans l'une des premières cellules (150), l'une des secondes cellules (160) ou l'un des connecteurs (121, 122).

9. Endoprothèse (100) selon l'une quelconque des revendications 1 à 8, comprenant en outre:
au moins un dispositif de maintien (170) conçu pour être accouplé un cathéter (200).

10. Endoprothèse (100) selon l'une quelconque des revendications 1 à 9, dans laquelle l'endoprothèse est fabriquée en Nitinol ou en acier inoxydable.

11. Procédé de production d'une endoprothèse (100), le procédé comprenant:
la sélection (510) d'un premier type de cellules (150);
la sélection (520) d'un second type de cellules (160);
la sélection (530) d'un premier type de connecteur (121); et
la fabrication (550) d'une endoprothèse (100) selon l'une quelconque des revendications 1 à 10 en disposant la pluralité des premières cellules (150) du premier type dans la première section (111) de l'endoprothèse (100), et en disposant la pluralité de secondes cellules (160) du second type dans la seconde section (112) de l'endoprothèse (100), et en reliant les cellules adjacentes (150) de la pluralité des premières cellules (150) au moyen d'une barre (130) aux coins respectifs formant un sommet (152) de chaque première cellule (150) dans la direction circonférentielle de l'endoprothèse (100), et en reliant les premières cellules (150) et les secondes cellules (160) au moyen du premier type de connecteur (121).

12. Procédé selon la revendication 11, dans lequel la fabrication (550) comprend:
la fourniture d'un matériau tubulaire pour l'endoprothèse (100); et
la découpe du matériau tubulaire selon le premier et le second type sélectionnés de cellules (150, 160) et du premier type de connecteur (121).

13. Procédé selon la revendication 11 ou 12, comprenant en outre:
la fourniture d'au moins un marqueur radioopaque (180); et
l'intégration de l'au moins un marqueur radioopaque (180) dans l'une des premières cellules (150), l'une des secondes cellules (160) et/ou l'un des connecteurs (121, 122).

14. Procédé selon l'une quelconque des revendications 11 à 13, comprenant en outre:
la formation d'au moins un dispositif de retenue (170) conçu pour être accouplé à un cathéter (200).

15. Procédé selon l'une quelconque des revendications 11 à 14, comprenant en outre:
la sélection (540) d'au moins un troisième type de cellules et/ou d'au moins un second type de connecteurs (122),
dans lequel la fabrication (550) comprend:
la disposition d'une pluralité de troisièmes cellules du troisième type dans une troisième section (113) de l'endoprothèse (100), et la liaison des secondes cellules (160) aux troisièmes cellules à l'aide du second type de connecteur (122), ou
la disposition d'une pluralité de troisièmes cellules du premier et second type dans une troisième section (113) de l'endoprothèse (100), et la liaison des secondes cellules (160) aux troisièmes cellules à l'aide du second type de connecteur (122), ou
la disposition d'une pluralité de troisièmes cellules du premier, second et troisième type dans une troisième section (113) de l'endoprothèse (100), et la liaison des secondes cellules (160) aux troisièmes cellules à l'aide du premier type de connecteur (122).
